# EUROPÄISCHE PATENTSCHRIFT

(11) **EP 0 451 793 B1**
(45) Veröffentlichungstag und Bekanntmachung des Hinweises auf die Patenterteilung: **13.09.1995**
(21) Anmeldenummer: 91105617.4
(22) Anmeldetag: 09.04.1991
(51) Int. Cl.: C07C 23/06, C07C 19/08, C07C 17/00, C07C 17/38, B01D 3/14

(54) **Verfahren zur Herstellung von Gemischen aus Chlortetrafluorethan und Octafluorcyclobutan**
Method for the production of mixtures of chlorotetrafluoroethane and octafluorocyclobutane
Procédé pour la fabrication de mélanges de chlorotétrafluoroéthane et d'octafluorocyclobutane

(30) Priorität: 12.04.1990 DE 4011820
(43) Veröffentlichungstag der Anmeldung: 16.10.1991
(73) Patentinhaber: HOECHST AKTIENGESELLSCHAFT, 65926 Frankfurt am Main (DE)
(72) Erfinder: Schöttle, Thomas, Dr., W-8263 Burghausen (DE); Weber, Herbert, W-8269 Burgkirchen (DE); Dostler, Werner, W-8261 Mettenheim-Hart (DE); Rettenbeck, Karl, W-8269 Burgkirchen (DE)

(56) Entgegenhaltungen:
- EP-A- 0 307 673
- US-A- 2 384 449
- US-A- 2 551 573
- US-A- 3 101 304
- DATABASE WPI Week 7750, Derwent Publications Ltd., London, GB; AN 77-89592Y & SU-A-541 835
- INDUSTRIAL & ENGINEERING CHEMISTRY RESEARCH Bd. 27, Nr. 1, Januar 1988, WASHINGTON US Seiten 208 - 211 EPHRAIM BROYER ET AL. 'Kinetics of the pyrolysis of Chlorodifluoromethane'

## Beschreibung

Die Erfindung betrifft ein Verfahren zur Herstellung von Gemischen aus Chlortetrafluorethan und Octafluorcyclobutan aus dem Gasstrom der Pyrolyse von Chlordifluormethan bei Temperaturen von oberhalb 600 °C, aus dem die überwiegende Menge an Chlorwasserstoff, Tetrafluorethylen (TFE), Hexafluorpropylen (HFP) und nicht umgesetztes Chlordifluormethan bereits entfernt worden sind, durch Destillation der verbliebenen Bestandteile in einer Rektifiziersäule.

Es ist bekannt, beispielsweise aus der US-PS 2 551 573, daß sich bei der Pyrolyse von Chlordifluormethan bei Temperaturen oberhalb von 600 °C neben dem Hauptprodukt TFE und neben Chlorwasserstoff noch weitere Fluor-, und Chlorfluoralkane, -cycloalkane und -alkene bilden, wie beispielsweise HFP, Octafluorcyclobutan, Chlortetrafluorethan, Chlorhexafluorpropan, Chlortrifluorethylen, Dichlordifluormethan und die isomeren Perfluorbutene. Daneben entstehen noch weitere Verbindungen, die höhere Siedepunkte aufweisen als die höchstsiedende der genannten Verbindungen, nämlich das Chlorhexafluorpropan. Die Perfluoralkene TFE und HFP besitzen eine große Bedeutung für die Herstellung von entsprechenden Fluorpolymeren und von Copolymeren aus diesen Monomeren. Octafluorcyclobutan und Chlortetrafluorethan und insbesondere Gemische dieser beiden Stoffe sind Ausgangsstoffe für die pyrolytische Darstellung von HFP, insbesondere nach einem Verfahren unter Zusatz von Tetrafluorethylen, wie es in der EP-OS 337 127 beschrieben ist.

Aus dem Gasstrom der Pyrolyse von Chlordifluormethan, die bei Temperaturen von 600 °C und darüber durchgeführt wird, werden zunächst die überwiegenden Anteile des Hauptproduktes TFE, des gebildeten Chlorwasserstoffs und des nicht umgesetzten Ausgangsproduktes Chlordifluormethan entfernt, zusammen mit allen Verbindungen, die tiefer als Chlordifluormethan (Kp -40,8 °C) sieden. Bei der destillativen Trennung des verbleibenden Produktgemisches werden nach einem Verfahren, wie es in der US-PS 3 101 304 beschrieben ist, zunächst die höher als Chlorhexafluorpropan siedenden Verbindungen (also mit Siedepunkten > +20 °C) am Fuß der Säule abgezogen. Der diese Säule über Kopf verlassende Anteil, der im wesentlichen HFP, Octafluorcyclobutan, Chlortetrafluorethan, Chlorhexafluorpropan, Chlortrifluorethylen und Dichlordifluormethan enthält, wird einer extraktiven Rektifizierung zugeführt. Als Extraktionsmittel werden aromatische Kohlenwasserstoffe sowie halogenhaltige aliphatische und aromatische Kohlenwasserstoffe eingesetzt. Auch Mineralöle sind geeignet (US-PS 2 384 449). Über Kopf dieser Säule werden dabei HFP und Octafluorcyclobutan als Gemisch abgeführt, dessen Trennung eine weitere Säule erfordert. Im Sumpfprodukt dieses Prozesses verbleibt ein Gemisch von im wesentlichen Chlortetrafluorethan, Chlorhexafluorpropan, Chlortrifluorethylen und Dichlordifluormethan zusammen mit dem Extraktionsmittel. Letzteres wird in einer weiteren Rektifiziersäule abgetrennt. Die weitere Auftrennung dieser das Chlortetrafluorethan enthaltenden Fraktion ist nicht beschrieben.

Es bestand daher die Aufgabe, ein einfaches Verfahren zur Gewinnung von Gemischen aus Chlortetrafluorethan und Octafluorcyclobutan zu entwickeln, bei dem vor allem der apparative Aufwand verringert werden kann.

Gemäß der vorliegenden Erfindung wird dies erreicht durch ein Verfahren der eingangs genannten Art, das dadurch gekennzeichnet ist, daß aus der Rektifiziersäule ein Gemisch von Chlortetrafluorethan und Octafluorcyclobutan als Seitenfraktion entnommen wird, wobei die Entnahme der Seitenfraktion in einem Bereich der Rektifiziersäule erfolgt, der 5 bis 90 % ihrer Gesamtlänge, gerechnet vom Kopf der Säule, umfaßt und wobei die Perfluorbutene und die höher als die Perfluorbutene siedenden Verbindungen im Sumpf der Rektifiziersäule verbleiben und die niedriger als Chlortetrafluorethan siedenden Verbindungen über Kopf abgeführt werden.

Das Ausgangsmaterial, das gemäß dem Verfahren der vorliegenden Erfindung behandelt wird, ist der Gasstrom der Pyrolyse des Chlordifluormethans, die bei einer Temperatur oberhalb von 600 °C durchgeführt wird, wobei aus diesem Gasstrom vorher nach bekannten Verfahren destillativ das gewünschte Hauptprodukt TFE, der bei der Pyrolyse anfallende Chlorwasserstoff sowie ferner HFP, das Ausgangsprodukt Chlordifluormethan und die tiefer als Chlordifluormethan siedenden Verbindungen entfernt worden sind. Demnach enthält dieses Ausgangsgemisch Chlortetrafluorethan, Octafluorcyclobutan, Chlortrifluorethylen, Dichlordifluormethan, die Perfluorbutene, Chlorhexafluorpropan und kleinere Anteile weiterer Verbindungen, die höher sieden als Chlorhexafluorpropan.

Unter "Perfluorbutene" sollen hier die beiden Perfluorbuten-Isomeren Perfluorbuten-(1) und Perfluorbuten-(2) verstanden werden, die gegebenenfalls auch etwas Perfluorisobutylen enthalten können. Unter "Chlortetrafluorethan" sind beide Isomeren 1-Chlor-1,1,2,2- und 1-Chlor-1,2,2,2-tetrafluorethan zu verstehen, wobei das erstere Isomere meist als überwiegender Bestandteil anzutreffen ist.

Gegebenenfalls können diesem Ausgangsgemisch noch andere Gase beigemischt werden, die im wesentlichen aus Chlortetrafluorethan oder Octafluorcyclobutan oder Gemischen dieser beiden Gase bestehen.

Dieses Ausgangsgemisch wird einer Rektifiziersäule zugeführt, die mit einer Einrichtung ausgestattet ist, die die Entnahme einer Seitenfraktion gestattet. Um einen ausreichenden Trenneffekt zu gewährleisten, soll diese Einrichtung im Bereich von 5 bis 90 % der Gesamtlänge der Säule, gerechnet vom Kopf der Säule, liegen, vorzugsweise in einem solchen Bereich von 20 bis 70 %.

Die Zuspeisung zur Rektifiziersäule erfolgt wie üblich von der Seite und soll im gleichen Bereich der Säule liegen wie die Entnahme. Vorzugsweise erfolgt die Zuspeisung in gleicher Höhe mit oder unterhalb der Entnahme der Seitenfraktion.

Der Druck in der Rektifiziersäule liegt bei Atmosphärendruck oder darüber und kann in weiten Grenzen schwanken. Zweckmäßigerweise hält man ihn, bei Verwendung von Wasser als Kühlmittel, bei 6 bis 8 bar.

Das als Seitenfraktion der Rektifiziersäule entnommene Gemisch hat eine Zusammensetzung von Chlortetrafluorethan : Octafluorcyclobutan im Bereich von 1 : 10 bis 10 : 1 Gew.-Teilen, vorzugsweise im Bereich von 1 : 4 bis 4 : 1 Gew.-Teilen.

Das über Kopf der Säule abgeführte Gemisch enthält die Verbindungen, die tiefer sieden als Chlortetrafluorethan, insbesondere Chlortrifluorethylen und Dichlordifluormethan. Im Sumpf der Rektifiziersäule finden sich neben Chlorhexafluorpropan und Verbindungen, die höher sieden als Chlorhexafluorpropan, insbesondere die Perfluorbutene.

Das erfindungsgemäße Verfahren bringt zunächst einen erheblichen apparativen Vorteil mit sich, da die Bestandteile Chlortetrafluorethan und Octafluorcyclobutan direkt im Gemisch anfallen und nicht in gesonderten Schritten von anderen Bestandteilen oder vom Extraktionsmittel getrennt werden müssen.

Ferner wird die nach der Extraktivdestillation schwierige Abtrennung der Perfluorbutene, die beim Octafluorcyclobutan verbleiben, hier problemlos möglich. Da beim erfindungsgemäßen Verfahren Octafluorcyclobutan und Chlortetrafluorethan im Gemisch vorliegen und dieses Gemisch einen azeotropen Punkt mit Siedepunktsminimum aufweist, wird dadurch ihre relative Flüchtigkeit gegenüber der der Perfluorbutene erhöht und damit deren Abtrennung erheblich verbessert.

Die Erfindung wird durch folgende Beispiele erläutert.

### Beispiel 1

Eine bei 7,5 bar betriebene Füllkörpersäule (42 Böden) von 21,4 m Länge und 0,3 m Durchmesser wird kontinuierlich mit 112,03 kg/h eines Gemisches beschickt, das folgende Komponenten enthält:

| | Gew.-% |
|---|---|
| Dichlordifluormethan | 0,43 |
| Chlortrifluorethylen | 1,29 |
| Rest 1 *) | 1,88 |
| Octafluorcyclobutan | 52,99 |
| Chlortetrafluorethan | 22,83 |
| Perfluorbuten-(2)_{}} | 2,22 |
| Perfluorbuten-(1)^{}} | |
| Chlorhexafluorpropan | 12,50 |
| Rest 2 **) | 5,85 |

| | |
|---|---|
| *) Rest 1: niedriger als Chlortetrafluorethan siedende Stoffe | |
| **) Rest 2: höher als die Perfluorbutene siedende Stoffe | |

Das Ausgangsgemisch wird an einem Punkt 83,4 % der Säulenlänge unterhalb des Kopfes zugespeist. Das Rücklaufverhältnis beträgt 1 : 10 bezüglich des Zulaufs der Säule. Die Temperatur der Säule reicht von 37 °C am Kopf bis 95 °C am Fuß der Säule.

Die Entnahme der Seitenfraktion, die überwiegend Octafluorcyclobutan und Chlortetrafluorethan enthält, erfolgt an einem Punkt 51,4 % der Säulenlänge unterhalb des Kopfes der Säule in einer Menge von 86,76 kg/h. Sie weist folgende Zusammensetzung auf:

| | Gew.-% |
|---|---|
| Dichlordifluormethan | 0,13 |
| Chlortrifluorethylen | 0,13 |
| Rest 1 *) | 0,63 |
| Octafluorcyclobutan | 67,70 |
| Chlortetrafluorethan | 29,29 |
| Perfluorbuten-(2)_{}} | 1,87 |
| Perfluorbuten-(1)^{}} | |
| Chlorhexafluorpropan | 0 |
| Rest 2 **) | 0,25 |

| | |
|---|---|
| *) Rest 1: niedriger als Chlortetrafluorethan siedende Stoffe | |
| **) Rest 2: höher als die Perfluorbutene siedende Stoffe | |

Die Seitenfraktion enthält 98 Gew.-% der Menge an Octafluorcyclobutan und 99,3 Gew.-% der Menge an Chlortetrafluorethan, bezogen auf die zugeführte Menge im Ausgangsgemisch.

### Beispiel 2

Es wird die im Beispiel 1 beschriebene Füllkörpersäule, ausgestattet mit der gleichen Zuspeisung benutzt. Die Entnahme der Seitenfraktion erfolgt an einem Punkt 39 % der Säulenlänge unterhalb des Kopfes der Säule. Sie wird betrieben mit einer Temperatur von 37,5 °C am Fuß und 93 °C am Kopf, das Rücklaufverhältnis ist wieder 1 : 10. Es werden kontinuierlich 124,07 kg/h eines Gemisches folgender Zusammensetzung zugespeist:

| | Gew.-% |
|---|---|
| Dichlordifluormethan | 0,41 |
| Chlortrifluorethylen | 1,64 |
| Rest 1 *) | 2,10 |
| Octafluorcyclobutan | 28,23 |
| Chlortetrafluorethan | 45,06 |
| Perfluorbuten-(2)_{}} | 2,93 |
| Perfluorbuten-(1)^{}} | |
| Chlorhexafluorpropan | 11,28 |
| Rest 2 **) | 8,34 |

| | |
|---|---|
| *) Rest 1: niedriger als Chlortetrafluorethan siedende Stoffe | |
| **) Rest 2: höher als die Perfluorbutene siedende Stoffe | |

Als Seitenfraktion werden 92,2 kg/h eines Gemisches folgender Zusammensetzung abgezogen:

| | Gew.-% |
|---|---|
| Dichlordifluormethan | 0,18 |
| Chlortrifluorethylen | 0,18 |
| Rest 1 *) | 0,89 |
| Octafluorcyclobutan | 37,07 |
| Chlortetrafluorethan | 60,09 |
| Perfluorbuten-(2)_{}} | 1,46 |
| Perfluorbuten-(1)^{}} | |
| Chlorhexafluorpropan | 0 |
| Rest 2 **) | 0,13 |

| | |
|---|---|
| *) Rest 1: niedriger als Chlortetrafluorethan siedende Stoffe | |
| **) Rest 2: höher als die Perfluorbutene siedende Stoffe | |

Die Seitenfraktion enthält 97,1 Gew.-% der Menge an Octafluorcyclobutan und 99,1 Gew.-% der Menge an Chlortetrafluorethan, bezogen auf die zugeführte Menge im Ausgangsgemisch.

## Patentansprüche

1. Verfahren zur Herstellung von Gemischen aus Chlortetrafluorethan und Octafluorcyclobutan aus dem Gasstrom der Pyrolyse von Chlordifluormethan bei Temperaturen von oberhalb 600 °C, aus dem die überwiegende Menge an Chlorwasserstoff, Tetrafluorethylen (TFE), Hexafluorpropylen (HFP) und nicht umgesetztes Chlordifluormethan bereits entfernt worden sind, durch Destillation der verbliebenen Bestandteile in einer Rektifiziersäule, dadurch gekennzeichnet, daß aus der Rektifiziersäule ein Gemisch von Chlortetrafluorethan und Octafluorcyclobutan als Seitenfraktion entnommen wird, wobei die Entnahme der Seitenfraktion in einem Bereich der Rektifiziersäule erfolgt, der 5 bis 90 % ihrer Gesamtlänge, gerechnet vom Kopf der Säule, umfaßt und wobei die Perfluorbutene und die höher als die Perfluorbutene siedenden Verbindungen im Sumpf der Rektifiziersäule verbleiben und die niedriger als Chlortetrafluorethan siedenden Verbindungen über Kopf abgeführt werden.

2. Verfahren zur Herstellung von Gemischen aus Chlortetrafluorethan und Octafluorcyclobutan aus dem Gasstrom der Pyrolyse von Chlordifluormethan bei Temperaturen von oberhalb 600 °C gemäß Anspruch 1, dadurch gekennzeichnet, daß die Entnahme der Seitenfraktion in einem Bereich der Rektifiziersäule erfolgt, der 20 bis 70 % ihrer Gesamtlänge, gerechnet vom Kopf der Säule, umfaßt.

3. Verfahren zur Herstellung von Gemischen aus Chlortetrafluorethan und Octafluorcyclobutan aus dem Gasstrom der Pyrolyse von Chlordifluormethan bei Temperaturen von oberhalb 600 °C gemäß Anspruch 1 und 2, dadurch gekennzeichnet, daß den verbliebenen Bestandteilen des Pyrolyse-Gasstroms noch andere Gase beigemischt werden, die im wesentlichen aus Chlortetrafluorethan oder Octafluorcyclobutan oder Gemischen dieser beiden Gase bestehen.

4. Verfahren zur Herstellung von Gemischen aus Chlortetrafluorethan und Octafluorcyclobutan aus dem Gasstrom der Pyrolyse von Chlordifluormethan bei Temperaturen von oberhalb 600 °C nach einem oder mehreren der Ansprüche 1 bis 3, dadurch gekennzeichnet, daß die entnommene Seitenfraktion eine Zusammensetzung von Chlortetrafluorethan : Octafluorcyclobutan im Bereich von 1 : 10 bis 10 : 1 Gew.-Teilen hat.

5. Verfahren zur Herstellung von Gemischen aus Chlortetrafluorethan und Octafluorcyclobutan aus dem Gasstrom der Pyrolyse von Chlordifluormethan bei Temperaturen von oberhalb 600 °C nach einem oder mehreren der Ansprüche 1 bis 4, dadurch gekennzeichnet, daß die entnommene Seitenfraktion eine Zusammensetzung von Chlortetrafluorethan : Octafluorcyclobutan im Bereich von 1 : 4 bis 4 : 1 Gew.-Teilen hat.

## Claims

1. A process for the preparation of mixtures of chlorotetrafluoroethane and octafluorocyclobutane from the gas stream from the pyrolysis of chlorodifluoromethane at temperatures above 600°C, from which the main amount of hydrogen chloride, tetrafluoroethylene (TFE), hexafluoropropylene (HFP) and unreacted chlorodifluoromethane have already been removed, by distillation of the remaining constituents in a rectifying column, which comprises withdrawing a mixture of chlorotetrafluoroethane and octafluorocyclobutane from the rectifying column as a side fraction, the withdrawal of the side fraction being carried out in a region of the rectifying column which includes 5 to 90% of its total length, calculated from the top of the column, and the perfluorobutenes and the compounds which boil at a higher temperature than the perfluorobutenes remaining in the bottom of the rectifying column and the compounds which boil at a lower temperature than chlorotetrafluoroethane being led off over the top.

2. The process for the preparation of mixtures of chlorotetrafluoroethane and octafluorocyclobutane from the gas stream from the pyrolysis of chlorodifluoromethane at temperatures above 600°C as claimed in claim 1, wherein the withdrawal of the side fraction takes place in a region of the rectifying column which includes 20 to 70% of its total length, calculated from the top of the column.

3. The process for the preparation of mixtures of chlorotetrafluoroethane and octafluorocyclobutane from the gas stream from the pyrolysis of chlorodifluoromethane at temperatures above 600°C as claimed in claims 1 and 2, wherein still other gases which essentially comprise chlorotetrafluoroethane or octafluorocyclobutane or mixtures of these two gases are admixed to the remaining constituents of the pyrolysis gas stream.

4. The process for the preparation of mixtures of chlorotetrafluoroethane and octafluorocyclobutane from the gas stream from the pyrolysis of chlorodifluoromethane at temperatures above 600°C as claimed in one or more of claims 1 to 3, wherein the side fraction withdrawn has a composition of chlorotetrafluoroethane: octafluorocyclobutane in the range from 1:10 to 10:1 parts by weight.

5. The process for the preparation of mixtures of chlorotetrafluoroethane and octafluorocyclobutane from the gas stream from the pyrolysis of chlorodifluoromethane at temperatures above 600°C as claimed in one or more of claims 1 to 4, wherein the side fraction withdrawn has a composition of chlorotetrafluoroethane:octafluorocyclobutane in the range from 1:4 to 4:1 parts by weight.

## Revendications

1. Procédé de préparation de mélanges de chlorotétrafluoroéthane et d'octafluorocyclobutane à partir du courant gazeux obtenu par la pyrolyse du chlorodifluorométhane à des températures supérieures à 600°C, dont l'essentiel du chlorure d'hydrogène, du tétrafluoroéthylène (TFE), de l'hexafluoropropylène (HFP) et du chlorodifluorométhane non transformé a déjà été éliminé, par distillation des constituants restants dans une colonne à rectification, caractérisé en ce qu'on prélève, comme fraction latérale, un mélange de chlorotétra-fluoroéthane et d'octafluorocyclobutane à partir de la colonne à rectification, le prélèvement de la fraction latérale ayant lieu dans un domaine de la colonne à rectification qui se situe entre 5 et 90% de sa longueur totale, calculée à partir de tête de la colonne, les perfluorobutènes ainsi que les composés ayant un point d'ébullition supérieur à ceux des perfluorobutènes restants au bas de la colonne de rectification et les composés ayant un point d'ébullition inférieur à celui du chlorotétrafluoroéthane sont éliminés à la tête de la colonne.

2. Procédé de préparation de mélanges de chlorotétrafluoroéthane et d'octafluorocyclobutane à partir du courant gazeux obtenu par la pyrolyse du chlorodifluorométhane à des températures supérieures à 600°C selon la revendication 1, caractérisé en ce que le prélèvement de la fraction latérale a lieu dans un domaine de la colonne de rectification qui se situe entre 20 et 70% de sa longueur totale, calculée à partir de la tête de la colonne.

3. Procédé de préparation de mélanges de chlorotétrafluoroéthane et d'octafluorocyclobutane à partir du courant gazeux obtenu par la pyrolyse du chlorodifluorométhane à des températures supérieures à 600°C selon les revendications 1 et 2, caractérisé en ce que d'autres gaz, qui sont constitués essentiellement de chlorotétrafluoroéthane ou d'octafluorocyclobutane ou de mélanges de ces deux gaz, sont mélangés encore aux constituants restants du courant gazeux de la pyrolyse.

4. Procédé de préparation de mélanges de chlorotétrafluoroéthane et d'octafluorocyclobutane à partir du courant gazeux obtenu par la pyrolyse du chlorodifluorométhane à des températures supérieures à 600°C selon l'une ou plusieurs des revendications 1 à 3, caractérisé en ce que la fraction latérale prélevée a une composition de chlorotétrafluoroéthane:octafluorocyclobutane comprise dans le domaine allant de 1:10 à 10:1 parties en poids.

5. Procédé de préparation de mélanges de chlorotétrafluoroéthane et d'octafluorocyclobutane à partir du courant gazeux obtenu par la pyrolyse du chlorodifluorométhane à des températures supérieures à 600°C selon l'une ou plusieurs des revendications 1 à 4, caractérisé en ce que la fraction latérale prélevée a une composition de chlorotétrafluoroéthane:octafluorocyclobutane comprise dans le domaine allant de 1:4 à 4:1 parties en poids.
